# EUROPEAN PATENT SPECIFICATION

(11) **EP 1 857 084 B1**
(45) Date of publication and mention of the grant of the patent: **21.10.2009**
(21) Application number: 07108391.9
(22) Date of filing: 16.05.2007
(51) Int. Cl.: A61F 9/007, F16K 31/122

(54) **Surgical system having pneumatic manifolds with integral air cylinders**
Chirurgisches System mit pneumatischen Übergangsstücken mit eingebauten Luftzylindern
Système chirurgical ayant des collecteurs pneumatiques avec des cylindres à air intégrés

(30) Priority: 19.05.2006 US 437301; 28.06.2006 US 477035
(43) Date of publication of application: 21.11.2007
(73) Proprietor: Alcon, Inc., 6331 Hünenberg (CH)
(72) Inventor: King, Nicolei, Aliso Viejo, CA California 82656 (US)
(74) Representative: Hanna, Peter William Derek

(56) References cited:
- EP-A- 1 091 130
- EP-A2- 0 095 926
- US-A- 4 758 238
- US-A- 5 107 899
- US-A- 5 549 139
- US-A1- 2004 195 537

## Description

### Background of the Invention

The present invention relates generally to surgical systems and more specifically to surgical systems that control pneumatic devices.

Many microsurgical procedures require precision cutting and/or removal of various body tissues. For example, certain ophthalmic surgical procedures require the cutting and/or removal of the vitreous humor, a transparent jelly-like material that fills the posterior segment of the eye. The vitreous humor, or vitreous, is composed of numerous microscopic fibers that are often attached to the retina. Therefore, cutting and removal of the vitreous must be done with great care to avoid traction on the retina, the separation of the retina from the choroid, a retinal tear, or, in the worst case, cutting and removal of the retina itself.

Conventional vitrectomy probes typically include a hollow outer cutting member, a hollow inner cutting member arranged coaxially with and movably disposed within the hollow outer cutting member, and a port extending radially through the outer cutting member near the distal end thereof. Vitreous humor is aspirated into the open port, and the inner member is actuated, closing the port. Upon the closing of the port, cutting surfaces on both the inner and outer cutting members cooperate to cut the vitreous, and the cut vitreous is then aspirated away through the inner cutting member. This cutting action may be made using an electric cutter, but pneumatically driven probes operating at a relatively high pressure are more common.

Additionally, during typical ophthalmic procedures, air may be introduced into the posterior chamber. This air must be of relatively low pressure.

Conventional ophthalmic surgical instrument systems use vacuum to aspirate the surgical site and positive pressure to irrigate the site. Typically, a cassette is serially connected between the means used to generate pressure and the surgical instrument. The use of cassettes with surgical instruments to help manage irrigation and aspiration flows at a surgical site is well known. Aspiration fluid flow rate, vacuum level, irrigation fluid pressure, and irrigation fluid flow rate are some of the parameters that require precise control during ophthalmic surgery. For aspiration instruments, the air pressure is below atmospheric pressure, and fluid is removed from the surgical site. For irrigation instruments, the air pressure is higher than atmospheric pressure, and the fluid will be transported from the irrigation fluid reservoir to the surgical site.

Prior art surgical systems have controlled the flow of irrigation and aspiration fluids using plunger-like pinch valves in the cassette operated by electric solenoids or pneumatic cylinders located within the surgical console (e.g. EP-A-0,095,926). For example, U.S. Patent No. 4,758,238 (Sundblom, et al.) discloses a disposable cassette having a plurality of fluid channels formed therein. Flow through these various fluid channels is controlled by a series of valve stem actuators 51 (see FIGS. 9 and 11, for example) that blocks flow in an associated fluid channels when driven by an externally mounted piston. While these systems are well-known in the art and work well, electric solenoids are problematic because of their size and substantial power requirements. Size is of a concern when such solenoids are to be incorporated into a relatively compact surgical console, and the power used by these solenoids requires a larger power source with the associated need to dissipate the additional noise and heat generated by the larger power source. In addition, electric solenoids do not apply a constant force over the entire length of movement of the solenoid piston, resulting in unpredictable sealing of the fluid channel. Prior art pneumatic cylinders must be supplied with pressurized air to work, and the associated air supply tubings used in prior art air cylinders require additional space within the control console as well as increasing the complexity of the system, as exemplified by US-A-5,549139 (Perkins et al.), resulting in increased manufacturing and maintenance costs. In addition, the air supply tubings can develop leaks over time, also increasing maintenance costs.

Accordingly, a need continues to exist for a surgical system having a simplified pinch valve actuation mechanism.

### Brief Summary of the Invention

The present invention improves upon prior art by providing a surgical system, in accordance with claims which follow, having all of the various pneumatic control sub-systems integrally mounted on a common manifold. The various required control mechanisms such as valves are likewise integrally mounted to the common manifold. Air cylinders for valve actuation are directly installed into the manifolds, and pressurized air for the air cylinders is supplied to the cylinders through the manifolds. Such a construction eliminated the need to use external air tubing to supply the air cylinders with pressurized air, thereby improving reliability and simplifying construction.

One objective of the present invention is to provide a surgical system having integrated pneumatic sub-systems.

Another objective of the present invention is to provide a surgical system having pneumatic sub-systems mounted on a common manifold.

Yet another objective of the present invention is to provide a surgical system for controlling pneumatic surgical devices.

Yet another objective of the present invention is to provide a surgical system for controlling pneumatic surgical devices having air cylinders for valve actuation directly installed into the pneumatic manifolds.

Still another objective of the present invention is to provide a surgical system for controlling pneumatic surgical devices having air cylinders for valve actuation directly installed into the pneumatic manifolds and pressurized air for the air cylinders is supplied to the cylinders through the manifolds.

These and other advantages and objectives of the present invention will become apparent from the detailed description, drawings and claims that follow.

### Brief Description of the Drawings

FIG. 1 is a front perspective view of a surgical console that may use the air cylinders of the present invention.
FIG. 2 is a front perspective view of a cassette that may be used with the air cylinders of the present invention.
FIG. 3 is a rear perspective view of a cassette that may be used with the air cylinders of the present invention.
FIG. 4 is an exploded perspective view of the integral pneumatics manifold that may be used with the air cylinders of the present invention.
FIG. 5 is a perspective view of the primary manifold of the present invention and illustrating several air cylinders of the present invention mounted on the manifold.
FIGS. 6a and 6b are perspective views of an air cylinder of the present invention.

### Detailed Description of the Preferred Embodiments

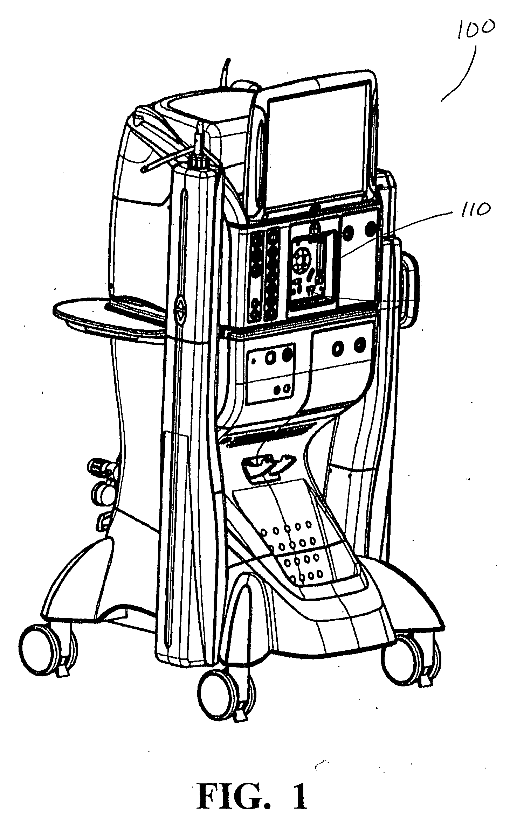

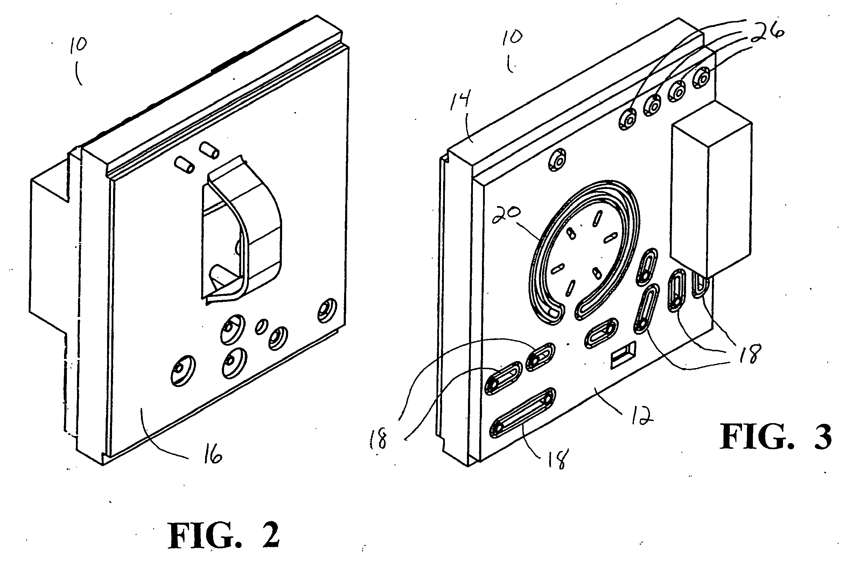

As best seen in FIGS. 1, 2 and 3, cassette 10 that may be used with the present invention generally included valve plate 12, body 14 and cover 16. Valve plate 12, body 14 and cover 16 may all be formed of a suitable, relatively rigid, and thermoplastic material. Valve plate 12 contains a plurality of openings 18 and pumping channel 20 that are sealed fluid tight by elastomers, forming a plurality of fluid paths. Ports 26 provide connectors between cassette 10 and surgical console 100 for the various irrigation and aspiration (pneumatic) functions of cassette 10 when cassette 10 is installed in cassette receiving portion 110 of console 100.

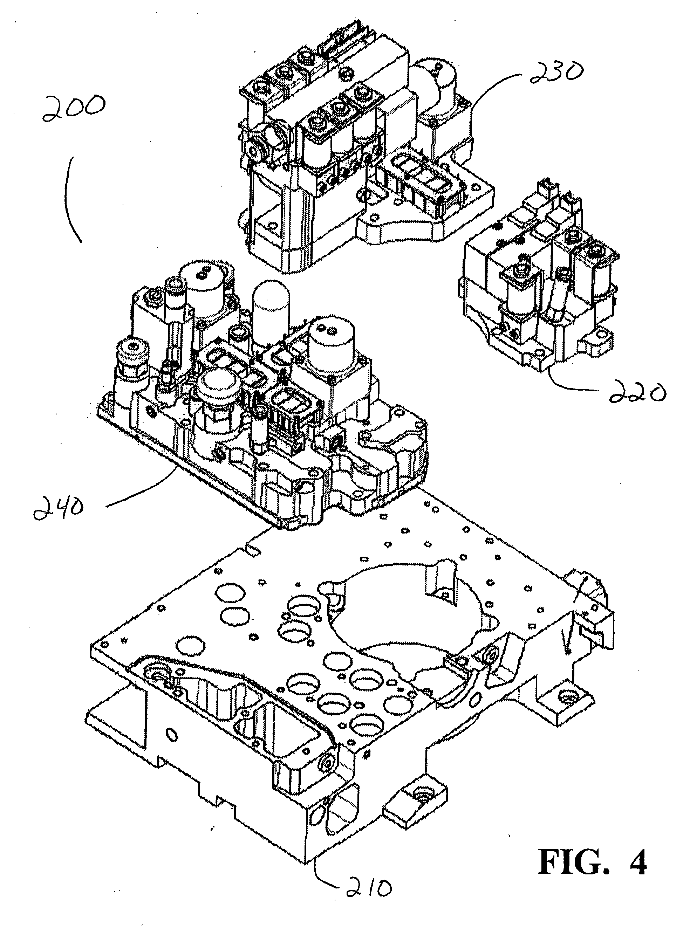

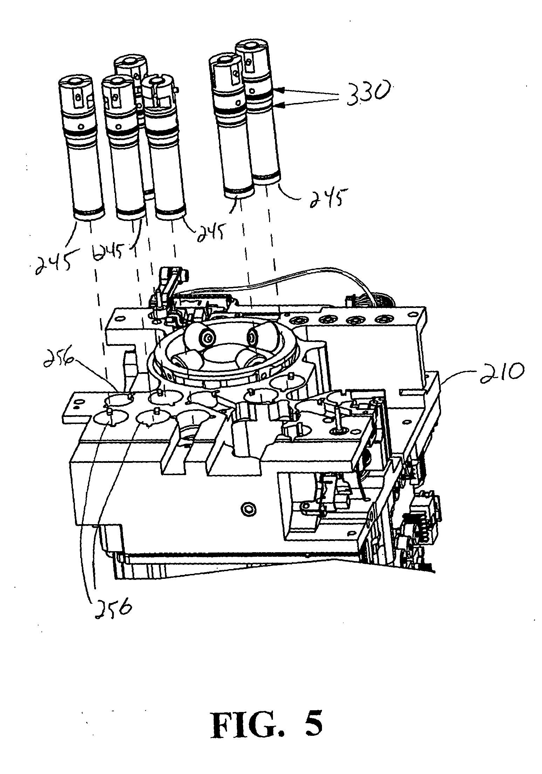

As best seen in FIG. 4, fluidics manifold 200 contains a plurality of subassemblies or manifolds mounted to common primary manifold 210. For example, fluidics manifold 200 may additionally contain aspiration manifold 220, and/or infusion/irrigation manifold 230 and/or valve or pincher manifold 240. As seen in FIG. 5, for example, each of manifolds 210, 220, 230 and 240 (manifold 240 used as an illustrative example) are self-contained, and may contain necessary the valves, regulators, sensors or other active embedded mechanical, electrical or electromechanical devices required to perform each manifold's primary function, such as air cylinders 245, by way of example. Manifolds 220 and 230 pneumatically and fluidly communicate with cassette 10 through primary manifold 210. Primary manifold 210 may be mounted in cassette receiving portion 110 of console 100 so that cassette 10 may be fluidly coupled to primary manifold 210. Primary manifold 210 may additionally contain pumps and fluid level and/or fluid flow sensors (all not shown).

Such a construction allows for the separation of the primary functionalities of each sub-assembly onto specific manifolds, thereby providing convenient and fast assembly, troubleshooting and repair. In addition, such a construction eliminates most of the various tubings and tubing connectors used in the prior art to connect the various components in each sub-assembly and reduces the overall size of the completed assembly.

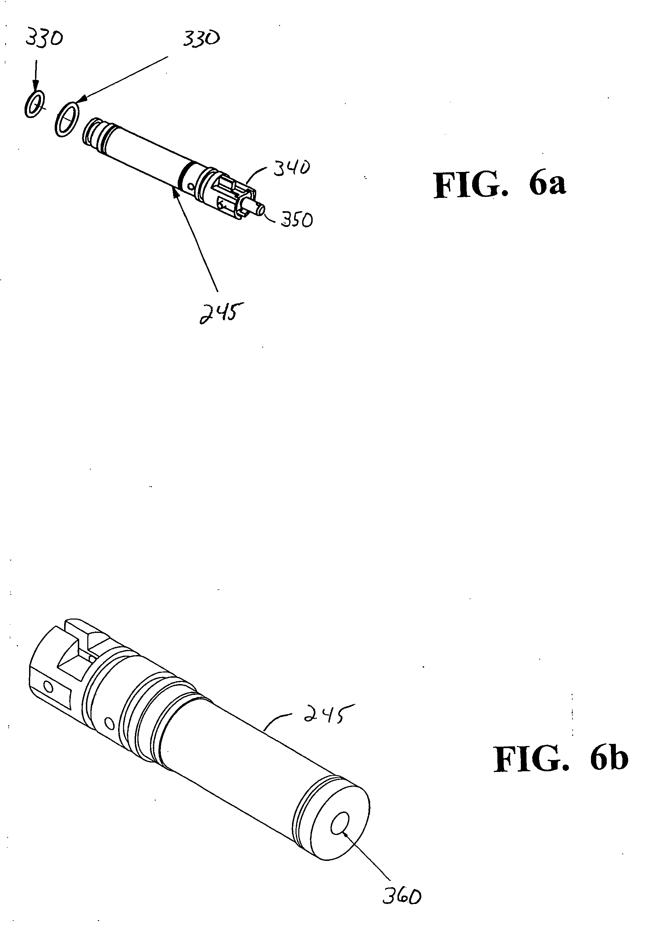

As best seen in FIGS. 5, 6a and 6b air cylinders 245 of the present invention are telescopically received within, for example, bores 256, in primary manifold 210, and sealed by O-rings 330. Exposed end 340 of cylinder 245 contains piston or plunger 350 that can be extended or retracted, as required, by pressurizing cylinder 245 to reciprocate through opening 18 in cassette 10 so as to operate a valve or other mechanism (not shown). Pressurized air is supplied to and vented from cylinder 245 through manifold 210, for example, via port 360 on cylinder 245 opposite piston 350. Such a construction eliminates the need for separate air supply lines to be run to cylinder 245, greatly simplifying the construction of console 100 and reducing the overall size of manifolds 210, 220, 230 and 240. In addition, as manifolds 210, 220, 230 and 240 may be precisely machined, cylinders 245 can be located very precisely.

This description is given for purposes of illustration and explanation. It will be apparent to those skilled in the relevant art that modifications may be made to the invention as herein described without departing from its scope.

## Claims

1. An ophthalmic surgical system comprising:
a surgical console (100) for managing irrigation and aspiration fluid flows by means of pneumatic sub-systems, the console comprising a cassette receiving portion (110) adapted to receive a cassette (10),
the system being **characterized by**;
a) at least one pneumatic manifold (200) located proximate to the cassette receiving portion (110) of the surgical console (100) so that the cassette may be fluidly coupled to the console, the manifold having at least one bore (256); and
b) an air cylinder (245) being telescopically received within the bore, the cylinder comprising a piston (350) and a port (360) pneumatically connecting the cylinder to the manifold, wherein the piston (350) is adapted to be extended or retracted by pressurized air supplied to the cylinder (245) through the manifold (210) and entering the cylinder through the port (360),
wherein the pneumatic manifold (200) comprises a primary manifold (210) together with an aspiration manifold (220) and/or an irrigation manifold (230) and/or a pincher manifold (240), wherein the aspiration manifold and the irrigation manifold are adapted to pneumatically and fluidly communicate with a cassette (10) through the primary manifold when the cassette is received by the cassette-receiving portion.

2. The surgical system of claim 1, further comprising a cassette (10) contained within the cassette receiving portion (110), the cassette containing an opening (18) through which the piston (350) may be reciprocated.

## Patentansprüche

1. Ophthalmisches chirurgisches System mit:
einer chirurgischen Konsole (100) zum Handhaben von Spül- und Saugfluidströmungen mittels pneumatischer Untersysteme, wobei die Konsole einen kassettenaufnehmenden Abschnitt (110), der zur Aufnahme einer Kassette (10) eingerichtet ist, aufweist; wobei das System **gekennzeichnet ist durch**:
a) zumindest einen pneumatischen Mehrfachverteiler (200), der nahe dem kassettenaufnehmenden Abschnitt (110) der chirurgischen Konsole (100) platziert ist, so dass die Kassette mit der Konsole in Fluidverbindung stehen kann, wobei der Mehrfachverteiler zumindest eine Bohrung (256) aufweist; und
b) einen Luftzylinder (245), der in der Bohrung teleskopisch aufgenommen ist, wobei der Zylinder einen Kolben (350) und eine Öffnung (360) hat, die den Zylinder mit dem Mehrfachverteiler pneumatisch verbinden, wobei der Kolben (350) eingerichtet ist, um **durch** dem Zylinder (245) über den Mehrfachverteiler (210) zugeführte und **durch** die Öffnung (360) in den Zylinder gelangende Druckluft ausgefahren oder eingezogen werden kann, wobei der pneumatische Mehrfachverteiler (200) einen primärer Mehrfachverteiler (210) zusammen mit einem Saug-Mehrfachverteiler (220) und/oder einem Spül-Mehrfachverteiler (230) und/oder einem Kneif("pincher")-Mehrfachverteiler (240) enthält, wobei der Saug-Mehrfachverteiler und der Spül-Mehrfachverteiler eingerichtet sind, um mit einer Kassette (10) über den primären Mehrfachverteiler pneumatisch und fluidmäßig zu kommunizieren, wenn die Kassette im kassettenaufnehmenden Abschnitt aufgenommen ist.

2. Chirurgisches System nach Anspruch 1, weiters mit einer Kassette (10), die im kassettenaufnehmenden Abschnitt (110) enthalten ist, wobei die Kassette eine Öffnung (18) aufweist, durch welche der Kolben (350) hin- und herbewegt werden kann.

## Revendications

1. Système chirurgical ophtalmique comprenant :
une console chirurgicale (100) pour gérer l'irrigation et l'aspiration d'écoulements de fluides au moyen de sous-systèmes pneumatiques, la console comprenant une partie de réception de cassette (110) adaptée pour recevoir une cassette (10),
le système étant **caractérisé par** :
a) au moins un collecteur pneumatique (200) situé à proximité de la partie de réception de cassette (110) de la console chirurgicale (100) de sorte que la cassette puisse être couplée fluidiquement à la console, le collecteur comportant au moins un alésage (256) ; et
b) un vérin pneumatique (245) reçu de manière télescopique dans l'alésage, le vérin comprenant un piston (350) et un orifice (360) reliant pneumatiquement le vérin au collecteur,
dans lequel le piston (350) est adapté pour être étendu ou rétracté par l'air sous pression délivré au vérin (245) par l'intermédiaire du collecteur (210) et entrant dans le vérin à travers l'orifice (360),
dans lequel le collecteur pneumatique (200) comprend un collecteur principal (210) ainsi qu'un collecteur d'aspiration (220) et/ou un collecteur d'irrigation (230) et/ou un collecteur d'étranglement (240), dans lequel le collecteur d'aspiration et le collecteur d'irrigation sont adaptés pour communiquer pneumatiquement et fluidiquement avec une cassette (10) par l'intermédiaire du collecteur principal lorsque la cassette est reçue par la partie de réception de cassette.

2. Système chirurgical selon la revendication 1, comprenant en outre une cassette (10) contenue dans la partie de réception de cassette (110), la cassette contenant une ouverture (18) à travers laquelle le piston (350) peut être déplacé en va-et-vient.
